Europäisches Patentamt

European Patent Office

Office européen des brevets

(11) Veröffentlichungsnummer: **0 231 425 B1**

(12) **EUROPÄISCHE PATENTSCHRIFT**

(45) Veröffentlichungstag der Patentschrift: 17.04.91

(51) Int. Cl.⁵: **A61K 37/547**

(21) Anmeldenummer: 86108530.6

(22) Anmeldetag: 23.06.86

(54) Verwendung einer Lösung von Alpha-Chymotrypsin.

(30) Priorität: 05.02.86 US 826337

(43) Veröffentlichungstag der Anmeldung:
12.08.87 Patentblatt 87/33

(45) Bekanntmachung des Hinweises auf die
Patenterteilung:
17.04.91 Patentblatt 91/16

(84) Benannte Vertragsstaaten:
AT BE CH DE FR GB IT LI LU NL SE

(56) Entgegenhaltungen:
FR-M- 3 739

CHEMICAL ABSTRACTS, Band 76, Nr. 23, 5.
Juni 1972, Seite 45, Zusammenfassung Nr.
135841q, Columbus, Ohio, US; C.A. WIND et
al.: "Effect of alpha-chymotrypsin on intraocular pressure and corneal wound healing",
& ANN. OPHTHALMOL. 1972, 4(1), 32-5, 39

BIOLOGICAL ABSTRACTS, Band 81, 1986,
Zusammenfassung Nr. 87308, Philadelphia,
PA, US; K.R. LITTLEWOOD et al.: "Vitreous
hemorrhage after cataract extraction", & BR.
J. OPHTHALMOL 69(2): 911-914, 1985

CHEMICAL ABSTRACTS, Band 67, Nr. 9, 28.

August 1967, Seite 3973, Zusammenfassung
Nr. 42496e, Columbus, Ohio, US; J. KRUDYSZ
et al.: "Experimental and clincal investigations on Wroclaw alpha-chymotrypsin", &
KLIN. OCZNA 37(1), 35-8(1967)

(73) Patentinhaber: Krumeich, Jörg H., Dr.
Propst-Hellmich-Promenade 28
W-4630 Bochum 6(DE)

(72) Erfinder: Krumeich, Jörg H., Dr.
Propst-Hellmich-Promenade 28
W-4630 Bochum 6(DE)

(74) Vertreter: Finkener und Ernesti Patentanwälte
Heinrich-König-Strasse 119
W-4630 Bochum 1(DE)

## Beschreibung

Die Erfindung betrifft die Verwendung einer Lösung von Alpha-Chymotrypsin im Rahmen der operativen Behandlung des Grauen Stars (grey cataract).

Der Graue Star ist eine Erkrankung des Auges, bei der es zu einer Trübung der Linse und einer dadurch verursachten Minderung der Sehfähigkeit kommt. Bei der sogenannten intrakapsulären Operation wird die getrübte Linse, die sich in einer Kapsel befindet, durch Extraktion als Ganzes zusammen mit der Kapsel entfernt.

Bei einer anderen Methode, der sogenannten extrakapsulären Operation, die man heute weitgehend anwendet, wird ebenfalls die trübe Linse entfernt, jedoch die hintere Kapsel belassen. Dabei geht man wie folgt vor: Zunächst wird die vordere Kapsel der Linse entfernt. Anschließend werden der Kern der Linse und die subkapsulären hinteren Rindenschichten mit Hilfe eines Spülverfahrens bei gleichzeitiger Saugung (irrigation, aspiration) abgesaugt. Es besteht auch die Möglichkeit, zur Zerstörung des Linsenkerns und zu dessen Absaugung ein entsprechend ausgebildetes Ultraschallgerät zu verwenden. Ziel dieses operativen Eingriffes ist es, die hintere Kapsel zu erhalten, damit eine klare Trennung zwischen Glaskörper und Vorderkammer aufrechterhalten wird und insoweit physiologische Verhältnisse erhalten bleiben. In den meisten Fällen wird bei derartigen Operationen die Einpflanzung einer Hinterkammerlinse appliziert, die die entfernte trübe Linse ersetzt. Voraussetzung hierfür ist, daß die hintere Kapsel bei der Operation erhalten wurde und daß die Kapsel völlig klar ist. Es ist bekannt, die hintere Kapsel vor der Einpflanzung der Hinterkammerlinse mechanisch zu polieren. Trotz ausgiebiger Politur kommt es häufig zu einer sogenannten Kapselfibrose; es handelt sich dabei um eine Verdickung der Kapsel aufgrund feinster Partikel, die mikroskopisch während der Operation nicht sichtbar sind und einen verdickten Film auf der Kapsel bilden.

Der Erfindung liegt die Aufgabe zugrunde, dies zu vermeiden und die Klarheit der hinteren Kapsel zu erhalten.

Es hat sich überraschenderweise herausgestellt, daß dies mit einer Lösung von Alpha-Chymotrypsin in einem physiologischen Lösungsmittel, insbesondere einer physiologischen Kochsalzlösung (Ringerlösung) oder einer Glutathion-Bicarbonat-Lösung nach Edelhauser in einer Verdünnung von mehr als 1 zu 10.000 bis zu 1 zu 30.000, vorzugsweise 1 zu 20.000 bis 1 zu 25.000 entsprechend einem Mengenverhältnis von 1 mg Alpha-Chymotrypsin zu mehr als 10 bis 30 ml, vorzugsweise 20 bis 25 ml des Lösungsmittels möglich ist. Bei Behandlung der hinteren Kapsel mit einer derartigen Lösung gelingt es, diese zu reinigen und die Eiweißbestandteile, die sich auf der Kapsel ansammeln und zu einem Film verdichten können, vollständig zu entfernen und auch deren Neubildung zu verhindern.

Versuche, die an Spenderaugen, deren Hornhäute für durchgreifende Hornhautübertragungen verwendet wurden, durchgeführt worden sind, haben gezeigt, daß von einer Lösung von Alpha-Chymotrypsin mit der oben angegebenen Verdünnung die Zonulafasern, die auch die hintere Kapsel festhalten, nicht aufgelöst werden, sondern daß ausschließlich die auf der Kapsel verbliebenen Restbestandteile entfernt werden.

Alpha-Chymotrypsin ist eine Substanz, die in reinster, kristallisierter, dyalysierter und stabilisierter Form im Handel erhältlich ist. Das Molekulargewicht beträgt 22.500, der isoelektrische Punkt liegt bei einem pH-Wert von 5 bis 4 und das Wirkungsoptimum bei 8,1 bis 8,6. Das Präparat kann in der oben angegebenen Verdünnung bei jeder extrakapsulären Cataract-Extraktion mit oder ohne Linsenimplantation zur Reinigung der hinteren Kapsel verwendet werden. Eine entsprechend verdünnte Lösung des Präparates kann von den im Handel erhältlichen Präparaten, die in Trockensubstanz angeboten werden, hergestellt werden.

Die Verwendung von Alpha-Chymotrypsin bei der Durchführung von intrakapsulären Operationen ist bekannt. Dabei wird die Substanz in einer Verdünnung von 1 zu 5.000 bis maximal 1 zu 10.000 verwendet und dient zur Zonulyse, d.h. zur Andauung der Zonulafasern, die die Linse umspannt halten. Es war daher überraschend festzustellen und nicht voraussehbar, daß die gleiche Substanz bei einer Verdünnung von mehr als 1 zu 10.000 bis 1 zu 30.000 die Zonulafasern nicht auflöst und zur Reinigung der hinteren Kapsel benutzt werden kann, wenn bestimmte Einwirkzeiten eingehalten werden.

Bei der Durchführung der extrakapsulären Operation hat es sich als zweckmäßig erwiesen, nach der in üblicher Weise erfolgenden Entfernung des trüben Linsenkerns und der subkapsulären Rindenschicht (Cortex) den entstandenen Hohlraum mit Luft zu füllen und mit einer Vorderkammerkanüle 0,1 bis 0,5 ml der erfindungsgemäßen verdünnten Lösung auf die hintere Kapsel aufzugeben. Nach einer ausreichenden Einwirkzeit wird die Lösung abgesaugt und anschließend gespült. Die Einwirkzeit ist in erster Linie abhängig von der Verdünnung der Lösung. Sie liegt für eine Lösung mit einer Verdünnung von 1 zu 25.000 bei etwa 2 1/2 Minuten. Lösungen mit einer Verdünnung von mehr als 1 zu 30.000 erfordern Einwirkzeiten, die den operativen Eingriff in unnötiger Weise verlängern würden. Bei der Verwendung von Lösungen mit einer unter 1 zu 15.000 liegenden Verdünnung würden sich einerseits Einwirkzeiten ergeben, die nicht mit der erforderlichen Sicherheit eingehalten werden können und es würde andererseits die Gefahr bestehen, daß die Zonulafasern zumindest teilweise aufgelöst oder angelöst werden. Der für die Praxis bevorzugte Bereich liegt bei einer Verdünnung von 1 zu 20.000 bis 1 zu 25.000 bei einer Einwirkzeit zwischen 2 und 3 Minuten.

Es ist auch möglich, die Lösung mittels eines schwammartigen Trägers zuzugeben, der mit 0,1 bis 0,5 ml der verdünnten Lösung getränkt, auf die hintere Kapsel aufgelegt und nach Ablauf der vorgesehenen Einwirkzeit entfernt wird.

**Ansprüche**

1. Lösung von Alpha-Chymotrypsin in einem physiologischen Lösungsmittel, insbesondere einer physiologischen Kochsalzlösung (Ringerlösung) oder einer Glutathion-Bicarbonat-Lösung nach Edelhauser in einer Verdünnung von mehr als 1 zu 10.000 bis 1 zu 30.000, vorzugsweise 1 zu 20.000 bis 1 zu 25.000, entsprechend einem Mengenverhältnis von 1 mg Alpha-Chymotrypsin zu mehr als 10 ml bis 30 ml, vorzugsweise 20 bis 25 ml des Lösungsmittels zur Behandlung und Reinigung der bei einer extrakapsulären Cataract-Operation belassenen hinteren Linsenkapsel.

2. Lösung nach Anspruch 1, dadurch gekennzeichnet, daß die Einwirkzeit einer Lösung mit einer Verdünnung von 1 zu 20.000 bis 1 zu 25.000 auf die hintere Linsenkapsel 2 bis 3 Minuten beträgt und für Lösungen mit geringerer Verdünnung verkürzt und bei Verwendung von Lösungen mit größerer Verdünnung verlängert wird.

Patentanspruch für folgenden Vertragsstaat: AT

1. Verwendung einer Lösung von Alpha-Chymotrypsin in einem physiologischen Lösungsmittel insbesondere einer physiologischen Kochsalzlösung (Ringerlösung) oder einer Glutathion-Bicarbonat-Lösung nach Edelhauser in einer Verdünnung von mehr als 1 zu 10.000 bis 1 zu 30.000, vorzugsweise 1 zu 20.000 bis 1 zu 25.000, entsprechend einem Mengenverhältnis von 1 mg Alpha-Chymotrypsin zu mehr als 10 ml bis 30 ml, vorzugsweise 20 bis 25 ml des Lösungsmittels zur Behandlung und Reinigung der bei einer extrakapsulären Cataract-Operation belassenen hinteren Linsenkapsel.

2. Verwendung einer Lösung nach Anspruch 1, dadurch gekennzeichnet, daß die Einwirkzeit einer Lösung mit einer Verdünnung von 1 zu 20.000 bis 1 zu 25.000 auf die hintere Linsenkapsel 2 bis 3 Minuten beträgt und für Lösungen mit geringerer Verdünnung verkürzt und bei Verwendung von Lösungen mit größerer Verdünnung verlängert wird.

**Claims**

1. Solution of alpha-chymotrypsin in a physiological solution medium, especially in a physiological saline solution or a solution of glutathionbicarbonate according to Edelhauser in a dilution of more than 1:10000 to 1:30000, preferably 1:20000 to 1:25000, corresponding to a ratio of amounts of 1 milligram of alpha-chymotrypsin to more than 10 to 30 milliliters, preferably 20 to 25 milliliters of the solution medium, for the treatment and cleaning of the posterior lens capsule left behind in an extracapsular cataract operation.

2. Solution according to claim 1, characterized in that the time of treatment of the posterior lens capsule is 2 to 3 minutes when using a solution with a dilution of 1:20000 to 1:25000 and is shortened when using solutions with a lower dilution and extended when using more diluted solutions.

Claims for the following Contracting State: AT

1. Use of a solution of alpha-chymotrypsin in a physiological solution medium, especially in a physiological saline solution or a solution of glutathion-bicarbonate according to Edelhauser in a dilution of more than 1:10000 to 1:30000, preferably 1:20000 to 1:25000, corresponding to a ratio of amounts of 1 milligram of alpha-chymotrypsin to more than 10 to 30 milliliters, preferably 20 to 25 milliliters of the solution medium, for the treatment and cleaning of the posterior lens capsule left behind in an extracapsular cataract operation.

2. Use of a solution according to claim 1, characterized in that the time of treatment of the posterior lens capsule is 2 to 3 minutes when using a solution with a dilution of 1:20000 to 1:25000 and is shortened when using solutions with a lower dilution and extended when using more diluted solutions.

**Revendications**

1. Solution d'alpha-chymotrypsine dans un solvant physiologique, notamment une solution de chlorure de sodium physiologique (solution de Ringer) ou une solution de glutathion-bicarbonate selon Edelhauser à une dilution de plus de 1 pour 10.000 à 1 pour 30.000, de préférence de 1 pour 20.000 à 1 pour 25.000, correspondant à une proportion de 1 mg d'alpha-chymotrypsine pour plus de 10 ml à 30 ml, de préférence de 20 à 25 ml du solvant pour le traitement et le nettoyage de la capsule de cristallin postérieure restant au cours de l'opération extracapsulaire de la cataracte.

2. Solution selon la revendication 1, caractérisée en ce que la durée d'action sur la capsule de cristallin postérieure d'une solution avec une dilution de 1 pour 20.000 à 1 pour 25.000 est de 2 à 3 minutes et que'elle est raccourcie pour des solutions avec une dilution plus faible et allongée par l'emploi de solutions avec une dilution plus forte.

Revendications pour l'Etat Contractant suivant: AT

1. Usage d'une solution d'alpha-chymotrypsine dans un solvant physiologique, notamment une solution de chlorure de sodium physiologique (solution de Ringer) ou une solution de glutathion-bicarbonate selon Edelhauser à une dilution de plus de 1 pour 10.000 à 1 pour 30.000, de préférence de 1 pour 20.000 à 1 pour 25.000, correspondant à une proportion de 1 mg d'alpha-chymotrypsine pour plus de 10 ml à 30 ml, de préférence de 20 à 25 ml du solvant pour le traitement et le nettoyage de la capsule de cristallin postérieure restant au cours de l'opération extracapsulaire de la cataracte.

2. Usage d'une solution selon la revendication 1, caractérisée en ce que la durée d'action sur la capsule de cristallin postérieure d'une solution avec une dilution de 1 pour 20.000 à 1 pour 25.000 et de 2 à 3 minutes et qu'elle est raccourcie pour des solutions avec une dilution plus faible et allongée par l'emploi de solutions avec une dilution plus forte.